# EUROPEAN PATENT APPLICATION

(11) **EP 4 711 378 A1**
(43) Date of publication of application: **18.03.2026**
(21) Application number: 24199968.9
(22) Date of filing: 12.09.2024
(51) Int. Cl.: C07K 7/06, C07K 14/78

(54) **PEPTIDE AGONISTS OF GPR56**

(71) Applicant: AcroPharma AB, 222 29 Lund (SE)
(72) Inventor: Salehi, Albert, 226 54 Lund (SE)
(74) Representative: KIPA AB

(57) **Abstract**

Disclosed are peptides that are useful as agonists of GPR56. Also disclosed are compositions comprising at least two synthetic or recombinant peptides, each of the peptides comprising a sequence selected from LLSSR, GYYCG, and NQGCKL, or a sequence that has a sequence identity of at least 60% thereto, wherein when a peptide comprises sequence a) or b) the peptide has a length of from 5-25 amino acids and wherein when a peptide comprises sequence c) the peptide has a length of from 6-26 amino acids. The compositions are useful for treating diseases or conditions such as vascular dysfunction, cardiovascular disease, impaired pancreatic β-cell function, type II diabetes, impaired glucose tolerance, depression, Alzheimer's disease, retinopathy and Attention deficit hyperactivity disorder.

## Description

### FIELD

The disclosure relates to peptide agonists, in particular peptide agonists of GPR56. The disclosure also relates to use of peptide agonists of GPR 56 for the treatment of disease.

### INTRODUCTION

G protein-coupled receptors (GPCRs) are the largest family of cell membrane proteins involving in signal transduction in different tissues in the human body. They are important players in almost all life activities and are responsible for regulating a cell's response to light, odor, hormones, neurotransmitters, chemokines, etc. The importance of GPCRs (almost more than 800 in humans) makes them one of the most important drug targets, playing a vital part in the development of drugs for major diseases such as cardiovascular disease, metabolic disease, neurological disease, immune disease, and cancer.

Human G protein-coupled receptor 56 (GPR56) is an adhesion GPCR that is encoded in humans by the gene ADGRG1 from chromosome 16q21 and is homologously encoded in mice at chromosome 8. Two splice forms, 687 and 693 amino acids in length are present in humans and mice. GPR56 has a 381 amino acid-long N-terminal extracellular segment and a GPCR proteolysis site upstream from the first transmembrane domain. GPR56 is mainly expressed in the heart, brain, thyroid, platelets, pancreatic β-cells and peripheral blood mononuclear cells. Accumulating evidence indicates that GPR56 promotes the formation of myelin sheaths and the development of oligodendrocytes in the cerebral cortex of the central nervous system. Moreover, GPR56 contributes to the development and differentiation of hematopoietic stem cells, induces adipogenesis, and regulates the function of immune cells. The lack of GPR56 leads to nervous system dysfunction, platelet disorders, and infertility. Metabolic disorders and cardiovascular diseases are associated with dysregulation of GPR56 expression, and reduced GPR56 activity is involved in the pharmacological resistance to some antidepressant and cancer drug treatments. Attributing to its significant biological functions and its long N-terminal extracellular region that interacts with multiple ligands, GPR56 is becoming an attractive therapeutic target.

Type 2 diabetes (T2D), also considered a metabolic disorder, is a disease where the body does not produce or properly use released insulin. Insulin is a hormone that is needed for the glucose uptake by the peripheral insulin sensitive tissues such as liver, certain muscle cells and adipose tissues, where glucose is stored or converted into energy needed for daily life of cells and tissues. The cause of T2D continues to be a mystery, although both genetics and environmental factors such as obesity and low-grade inflammation as well as the lack of exercise appear to play roles.

While the number of T2D patients worldwide has been estimated to 380 million, the number of pre-diabetic subjects are increasing and have been estimated to 318 million.

T2D and its devastating cardiovascular complications usually develops as a sequel of obesity and metabolic disorder, when the insulin secretion from the pancreatic beta-cells cannot adapt to the increased insulin requirements of the peripheral organs.

Metabolic disturbance such as long-lasting hyperglycemia and low-grade inflammation is a putative causal risk factor for development of cardiovascular and coronary artery disease in obesity and diabetes. The intrinsic link underlying vascular dysfunction during the mentioned conditions is not fully understood.

There is currently no effective treatment for prevention of type 2-diabetes (T2D) which is prompted by β-cell dysfunction, and its associated cardiovascular complications, which are prompted by endothelial cell (EC) dysfunction. The β-cell and EC failure precipitates T2D in insulin-resistant obesity. β-cell and EC dysfunction results from glucotoxic and associated pathophysiological episodes such as low-grade inflammation during the years of prediabetes.

GPR56 has been identified as a potential drug target for both neurological and psychiatric disorders where deficiency in GPR56 activity is thought to be associated with disease progression. Therapeutic solutions based on GPR56 targeting remain however elusive.

### SUMMARY

The present disclosure seeks to provide solutions to overcome the foregoing limitations or disadvantages of the prior art.

The present disclosure thus provides certain peptides that have therapeutic activity that is mediated by their ability to bind to GPR56 and thereby stimulate its activity. The peptides are thus agonists of GPR56.

In an aspect, the disclosure relates to a composition comprising at least two synthetic or recombinant peptides, each of the peptides comprising a sequence selected from LLSSR (SEQ ID NO:1), GYYCG (SEQ ID NO:2), and NQGCKL (SEQ ID NO:3), or a sequence that has a sequence identity of at least 60% thereto, wherein when a peptide comprises sequence a) or b) the peptide has a length of from 5-25 amino acids and wherein when a peptide comprises sequence c) the peptide has a length of from 6-26 amino acids.

The peptides can be selected from peptides having the general formula:

R₁-X₁X₂SSR-R₂

R₃-X₃YYX₄X₅-R₄, and

R₅-NQGCKX₆-R₆;

wherein
R₁, R₂, R₃, R₄, R₅ and R₆ are independently absent or a peptide sequence of 1-10 amino acids,
X₁, X₂ and X₆ are independently selected from L, A, V, I and G
X₃ and X₅ are independently selected from G and A; and
X₄ is selected from G, A and C.

The peptides can independently contain one or more modification. The modification can be selected from: acetylation at the N terminus; amidation at the C terminus; alkylation of at least one nitrogen atom; thionation of at least one carbonyl group; glycosylation and/or PEGylation.

Additionally or alternatively, the peptides can be cyclical and/or contain one or more D-amino acid.

The peptides can preferably contain one or more of the sequences LLSSR, GYYCG and NQGCKL. For example, the composition can contain two or more peptides, where the peptides each contains one or more of the sequences LLSSR, GYYCG and NQGCKL. The composition can contain two or more peptides, where each of the peptides contains one of the sequences LLSSR, GYYCG and NQGCKL. The composition can contain three peptides, where the peptides each contains one of the sequences LLSSR, GYYCG and NQGCKL. The three sequences LLSSR, GYYCG and NQGCKL can thus be present in separate peptides in the composition.

One or more of the peptides can contain or consist of a subsequence of the sequence of human collagen III. For example, two of the peptides can contain or consist of a subsequence of the sequence of human collagen III (SEQ ID NO:4). In particular, the peptides can comprise a peptide that is a subsequence, of the sequence defined by residues 1282 to 1287 in SEQ ID NO: 4; and/or a peptide that is a subsequence of the sequence defined by by residues 1359 to 1363 in SEQ ID NO:4.

The peptides and/or compositions containing the peptides, can be useful for treating human diseases or conditions that are manifested by impaired GPR56 function. The disease or conditions can be selected from vascular dysfunction, cardiovascular disease, impaired pancreatic b-cell function, diabetes, pre-diabetes, metabolic syndrome, depression, Alzheimer's disease, retinopathy and Attention deficit hyperactivity disorder.

Accordingly, the disclosure also relates to a peptide or composition as disclosed herein for use in the treatment of a disease or condition selected from vascular dysfunction, cardiovascular disease, impaired pancreatic b-cell function, diabetes, pre-diabetes, metabolic syndrome, depression, Alzheimer's disease, retinopathy and Attention deficit hyperactivity disorder.

The disclosure also relates to the treatment of a disease or condition selected from vascular dysfunction, cardiovascular disease, impaired pancreatic b-cell function, diabetes, prediabetes, metabolic syndrome, depression, Alzheimer's disease, retinopathy and Attention deficit hyperactivity disorder. The method preferably comprises administering to a human subject in need thereof a therapeutically effective amount of at least two synthetic or recombinant peptides as disclosed herein. Each of the peptides preferably comprising a sequence selected from LLSSR, GYYCG, and NQGCKL, or a sequence that has a sequence identity of at least 60% thereto, wherein when a peptide comprises sequence a) or b) the peptide has a length of from 5-25 amino acids and wherein when a peptide comprises sequence c) the peptide has a length of from 6-26 amino acids.

Additional features and advantages are described, and will be apparent from, the following detailed description and the figures.

### BRIEF DESCRIPTION OF THE DRAWINGS

FIG. 1 shows a diagram of the proposed mechanism of action of peptide agonists of GPR56
FIG. 2 shows (A) the dose-response effect of synthesized peptides on insulin secretion and (B) the cAMP content in human pancreatic insulin cells (EndoC bH1) cells.
FIG. 3 shows the dose-response effect of synthesized peptides on (A) the insulin secretion and (B) cAMP content in rat pancreatic insulin cells.
FIG. 4 shows the dose-response effect of synthesized peptides on the cAMP content in a human endothelial cell line (HUVEC).
FIG. 5 shows effect of peptide mixture on cell viability in HUVEC cells, where in (A) the effect is shown in the presence and absence of LPS (1000 ng/mL) and in (B) in the presence of low (5mM) and high (33mM) glucose respectively.
FIG. 6 shows tube formation assay on HUVEC cells in the presence of glucose, with and without peptide mixture (5G glucose shown in (A) and (C), 20G in (B), (D) and enlarged image in (E)).
FIG. 7 shows tube formation assay on HUVEC cells in the presence of normal (5G) glucose (A), glucose and LPS (B), with the effect of peptide mixture shown in (C) and (D).
FIG. 8 shows results of cell viability assay using INS-1 832/13 cells in the presence of peptide A (LLSSR), peptide B (NQGCKL) or peptide C (GYYCG) alone or in combinations for 24 hours, with cellular viability measured by MTS.

### DESCRIPTION

The present disclosure originates in the observation that prevention of normal physiological function of GPR56 is associated with cell dysfunctionality in insulin releasing pancreatic β-cells and in endothelial cells. Previous studies have found that metabolic disorder such as hyperglycemia, hyperlipidemia as well as presence of inflammatory cytokines, negatively influences the expressional level of GPR56.

Certain peptide sequences have been found to have agonist activity for GPR56, and thus have therapeutic potential through their ability to increase and/or rescue deficient GPR56 function.

The peptides were developed based on a structural comparison and computational analysis of the interaction between GPR56 and collagen type III, and through *de novo* design to mimic the binding of GPR56 residues to collagen III. Through this design, peptides that mimic the natural interaction of collagen type III and GPR56 were therefore developed, the peptides thereby having therapeutic activity.

One or more of the peptides in accordance with the present disclosure can contain or consist of a subsequence of human collagen III (SEQ ID NO:4). Thus one or more of the peptides can be selected from
i. a peptide that is a subsequence of the sequence defined by residues 1272 to 1297 in SEQ ID NO:4; and
ii. a peptide that is a subsequence of the sequence defined by by residues 1349 to 1373 in SEQ ID NO:4.

One of the peptides, recited in (i) can contain the sequence NQCGKL and another of the peptides, recited in (ii) can contain the sequence LLSSR.

Human G-protein coupled receptors (GPCRs) are the most abundant cell surface protein in human tissues regulating a diverse array of physiological/pathophysiological function. Analysis of the expressional level of all GPCRs in human endothelial cells of different origin has resulted in GPR56 and GPR126 being found among the highly expressed GPCRs in human umbilical vein endothelial cells (HUVEC), human coronary artery endothelial cells (CAEC), human aortic endothelial cells (HAEC) and human microvascular endothelial cells (MVEC).

As disclosed herein, "GPR56" refers to a G protein-coupled receptor 56 protein, which is sometimes also referred to as BFPP, BPPR, CDCBM14B, CDCBM15A, GPR56, TM7LN4 or TM7XN1 and is encoded in humans by the ADGRG1 gene.

A comparative study on the structure of GPR56 and its naturally occurring ligand collagen type III (Coll III) resulted in the development of peptides with short amino acid sequences as agonists for GPR56 activation. The peptides have been shown to rescue GPR56 deficiency, as manifested by the experimental data provided herein. These peptides are therefore useful in the prevention/treatment of certain human diseases that are manifested by defective GPR56 function, such as vascular dysfunction to prevent cardiovascular plaque formation in cardiovascular disease, deficient β-cell function and in neurodegenerative and neurological diseases such as Alzheimer disease, multiple sclerosis and ADHD.

The peptides are thus believed to have activity to prevent and/or ameliorate cardiovascular disease, in particular cardiovascular disease that is caused by, or accompanied by, endothelial cell dysfunction. The peptides can also be used for the improvement of the impaired pancreatic β-cell function in prediabetic and diabetic subjects as well as prevention of diabetic cardiovascular complications.

Cardiovascular disease in the present context can be selected from the group consisting of cardiac ischemia, stroke, heart failure, hypertensive heart disease, rheumatic heart disease, cardiomyopathy, heart arrhythmia, congenital heart disease, valvular heart disease, carditis, aortic aneurysms, peripheral artery disease, thromboembolic disease, and venous thrombosis.

Although previous studies have emphasized the beneficial effects of GPR126 stimulation on the cardiovascular function, the present inventor has found that GPR56 expression/activation has a regulatory impact on the GPR126 expression thus highlighting the importance of GPR56 for a proper cardiovascular function. It has also been found that down-regulation of GPR56 is associated with an increased inflammatory signals and reduced eNOS expression which is mediated pulsatile NO production and blood vessels dilution (reducing high blood pressure) as well as an increased endothelial apoptosis.

Furthermore, experimental in vitro studies on the endothelial cells have shown that GPR56 expression/activation play an extremely important role in endothelial tube formation.

The importance of GPR56 for the vascular endothelial cell function is highlighted by the observation that knockdown of GPR56 (GPR56-KD) is associated by a markedly reduced cell viability. Since previous investigations have revealed the activation of protein kinase A (PKA) downstream of cAMP generation via GPR56 activation, the present disclosure compares the effect of a peptide mixture to known PKA activators (Sp-8-Br-cAMP and Bt₂cAMP), PKA inhibitor (Rp-8-Br-cAMP) and collagen III (Col III), as described herein in the following Examples. Although the presence of LPS during culture-period was found to reduce cell viability, the effect was prevented when PKA activators, Coll III or a mixture of agonist peptides as disclosed herein were present during culture period. This indicates that activation of GPR56 counteracts the unbeneficial effect of LPS on cell viability. The selectivity of the developed peptides for GPR56 was confirmed by the observation that GPR56-KD attenuated the beneficial effect of Coll III and peptide-mixture to the same magnitude in cultured HUVEC cells.

It therefore follows that the peptides disclosed herein that mimic the interaction of of collagen III (Coll III) with GPR56 are predicted to have therapeutic effects, in particular in relation to deficient endothelical cell function and/or deficient pancreatic beta cell function.

GPR56 is the most abundantly expressed aGPCR in β cells of human and murine islets, an indication of its importance for islet function. It has been found that GPR56 mRNA expression in human islets is negatively correlated to β-cell dysfunction and risk of type II diabetes. Additionally, GPR56 has been found to promote insulin secretion and protect against cytokine-induced β-cell apoptosis when activated by endogenous collagen III.

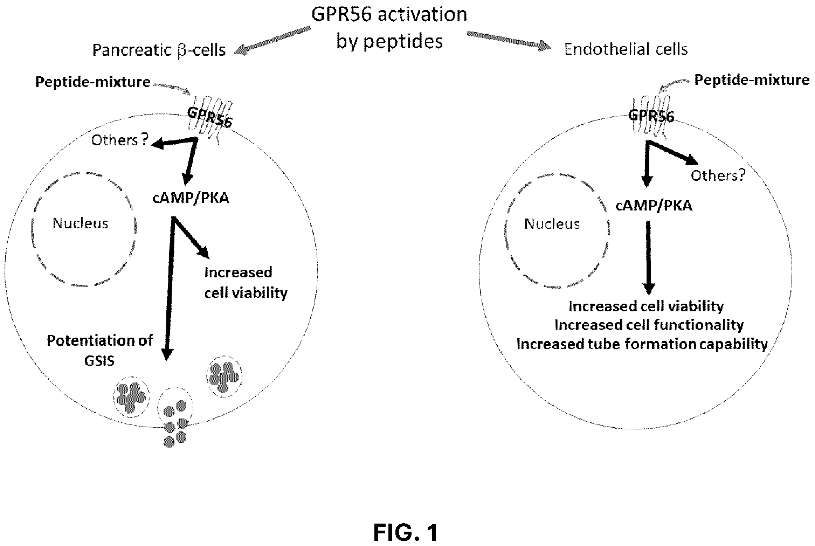

Peptide agonists of GPR56 are proposed to result in potentiation of therapeutically meaningful responses depending on the target tissue, as shown in FIG. 1. Thus in pancreatic β-cells (left), the binding of peptides mimicking the action of collagen III results in the intracellular stimulation of cAMP/PKA pathway. This stimulation on one hand results in increased cell viability and on the other hand potentiates glucose stimulated insulin secretion (GSIS), thereby ameliorating deficient insulin secretion that is one of the hallmarks of type 2 diabetes.

The therapeutic peptide agonists therefore can have activity for preventing the progress of diabetes, in particular type II diabetes, and/or preventing progress of prediabetes to diabetes and/or to stimulate the reversal of diabetes, and to prevent progression of cardiovascular complications due to hyperglycemia and/or low grade inflammation. In a particular embodiment the peptide agonists may have activity for reversal of diabetes due to reversal of beta cell dysfunction.

Treatment of diabetes can comprise one or more of: inducing glucose-stimulated insulin secretion; improving glucose tolerance; restoring insulin secretion from pancreatic β-cells in a subject affected by diabetes; and prevention of β-cell dysfunction.

The peptides are contemplated to additionally and/or alternatively have activity to prevent progression of prediabetes to diabetes, including type II diabetes.

Successful treatment of diabetes can be manifested by one or more of: inducing glucose-stimulated insulin secretion; improving glucose tolerance; restoring insulin secretion from pancreatic β-cells of a subject affected with diabetes; and prevent β-cell dysfunction.

Retinopathy is manifested by mitochondrial instability and mitochondrial dysfunction. Additionally, mitochondrial dysfunction can predispose to development of diabetes. Mitochondrically located PKA is furthermore known to reverse mitochondrial dysfunction. The stimulation of GPR56 increases PKA activity intracellularly, thereby resulting in prevention and/or amelioration of mitochondrial dysfunction. Thereby, the agonist peptides of GPR56 disclosed herein are expected to have therapeutic activity to treat or prevent retinopathy and other diseases and conditions that manifest through mitochondrial dysfunction.

Endothelial cells are important constituents of blood vessels that play critical roles in cardiovascular homeostasis by regulating blood fluidity and fibrinolysis, vascular tone, angiogenesis, monocyte/leukocyte adhesion and platelet aggregation. The normal vascular endothelium is taken as a gatekeeper of cardiovascular health, whereas abnormality of vascular endothelium is a major contributor to a plethora of cardiovascular ailments, such as atherosclerosis, aging, hypertension, obesity, and diabetes. Endothelial dysfunction is characterized by imbalanced vasodilation and vasoconstriction, elevated reactive oxygen species (ROS), and proinflammatory factors, as well as deficiency of nitric oxide (NO) bioavailability. The occurrence of endothelial dysfunction disrupts the endothelial barrier permeability that is a part of inflammatory response in the development of cardiovascular diseases. As such, abrogation of endothelial cell activation/inflammation is of great clinical relevance.

In endothelial cells, stimulation of GPR56 potentiates the cAMP/PKA pathway, resulting in increased cell viability and increased cell function. A consequence is the improved tube formation capabilities of the cells, a measure of their functional capabilities. Consequently, the peptides disclosed herein have therapeutic activity in the treatment of disorders manifested by deficient endothelial cell function, in particular cardiovascular diseases.

As shown in a study by Belzeaux (Nature Comm 2020, 11:1635), patients with depression who respond to treatment with serotonin-norepinephrine reuptake inhibitors have elevated levels of GPR56 mRNA. The study also shows that individuals who die by suicide have decreased levels of GPR56 mRNA implicating the importance of GPR56 activation in the treatment of depression. Furthermore, the authors showed that a tethered peptide agonist of GPR56 has antidepressant activity.

As a consequence, the peptides disclosed herein, which are able to rescue deficient GPR56 activity, are expected to have antidepressant activity through their ability to increase GPR56 activity in vivo. Through the activation of GPR56, the peptides can also improve the outcome of therapy using serotonin-norepinephrine reuptake inhibitors, by minimizing the need for the inhibitors.

GPR56 has been found to be important for brain development and function, including cerebral cortex (reviewed in Su et al, Acta Physiologica 2022, 00:e13866)). The protein is abundantly expressed in microglia and has been suggested to be important for macrophage infiltration in pathological conditions. The interaction of collagen III and GPR56 triggers G_{α12/13}-RhoA signaling, ensuring the formation of proper cortical lamination by inhibiting the overmigration of neurons, a manifestation of the importance of the protein in human cortex.

Mutations in GPR56 are the cause of a genetic disorder of human cortex development, namely BFPP (bilaterial frontoparietal polymicrogyria). The protein has also been implicated in Alzheimer's disease and multiple sclerosis. In particular, the role of GPR56 in microglial cells, the protein is proposed to be important for neurinflammation, which is important in both Alzheimer's disease and multiple sclerosis.

The peptides described herein therefore can act to activate GPR56 to prevent neuroinflammation in neurodegenerative disease such as Alzheimer's disease and multiple sclerosis.

GPR56 has also been implicated in psychiatric and neurodevelopmental diseases, including Bilateral frontoparietal polymicrogyria (BFPP), depression, epilepsy, autism and ADHD, which is consistent with its expression in tissues important for brain development, including cortex, hippoxampus, hypothalamus and amygdala (reviewed by Qi (Biochem Pharmacol 2024, 226:116395)).

Accordingly, due to their ability to ameliorate and/or rescue deficient GPR56 signaling activity, the peptides disclosed herein are contemplated to be useful in the treatment of human psychiatric and neurodevelopmental diseases, including Bilateral frontoparietal polymicrogyria (BFPP), depression, epilepsy, autism and/or ADHD.

The sequence NQGCKL (SEQ ID NO:3) corresponds to positions 1282 to 1287 and the sequence LLSSR (SEQ ID NO:1) corresponds to positions 1359 to 1363 in the human collagen III sequence (SEQ ID NO:4). These sequences have been found to be particularly important for GPR56 interaction, leading to the development of peptide sequences that mimic this interaction.

A third peptide comprising the sequence GYYCG (SEQ ID NO:2) has been developed as a novel GPR56 interacting molecule that also serves as an agonist of GPR56 function.

The peptides useful for ameliorating GRP56 deficiency threefore include peptides that contain one or more of the amino acid sequences LLSSR, GYYCG and NQGCKL and peptide sequences that have a sequence identity of at least 60% to these peptides. The peptides can generally have a length of 5-20 amino acids, except that in the case of peptides including the sequence NQGCKL or sequences at least 60% identical the peptides generally have a length of 6-26 amino acids.

Any combination of at least one of, and preferably at least two of the disclosed peptides is predicted to have therapeutic function through their agonistic activitation of GPR56.

The peptides having therapeutic potential can generally be selected from peptides that contain one or more of the sequences LLSSR, GYYCG and NQGCKL, or a sequence that has a sequence identity of at least 60% thereto, the peptides having a length in the range of 5 - 25 amino acids when containing the sequence LLSSR or GYYCG or a length in the range of 6 - 26 amino acids when containing the sequence NQGCKL. The peptides can have up to 10 amino acid residues flanking one or both of the sequences LLSSR, GYYCG and NQGCKL, or sequences with a sequence identity of at least 60% thereto.

The peptides can preferably contain two or more of the sequences LLSSR, GYYCG and NQGCKL, or a sequence that has a sequence identity of at least 60% thereto.

The peptides can have the general formula:

R₁-X₁X₂SSR-R₂

R₃-X₃YYX₄X₅-R₄, and

R₅-NQGCKX₆-R₆;

wherein
R₁, R₂, R₃, R₄, R₅ and R₆ are independently absent or a peptide sequence of 1-10 amino acids,
X₁, X₂ and X₆ are independently selected from L, A, V, I and G
X₃ and X₅ are independently selected from G and A.
X₄ is selected from G, A and C.

A peptide mixture with therapeutic potential can preferably be selected from a mixture comprising any combination of at least two peptides that have the sequence LLSSR, GYYCG or NQGCKL. Specifically, the peptide mixture can comprise a combination or mixture of the peptides (i) LLSSR and GYYCG; (ii) LLSSR and NQGCKL; (iii) GYYCG and NQGCKL; (iv) LLSSR; GYYCG and NQGCKL.

A therapeutic peptide, used alone or in combination with other peptides, can be selected from LLSSR, GYYCG and NQGCKL, or a peptide with at least a 60% sequence identity thereto.

In preferred embodiments a therapeutic peptide is a peptide selected from LLSSR, GYYCG and NQGCKL.

The peptide disclosed herein can have a binding affinity to a GPR56 protein to stimulate the activity thereof. The measured binding affinity of individual peptides in the peptide compositions disclosed herein to GPR56 can be less than (i.e. stronger than) 100 µM , such as less than 50 µM , such as less than 10µM, such as less than 5µM, less than 1µM or less than 100nM.

Preferably the therapeutic peptides disclosed herein do not have a binding affinity to other proteins, in particular the peptides do not have a binding affinity to other GPR proteins.

Binding affinity can be measured using methods known in the art. For example, binding affinity can be measured using an assay with labelled peptide or alternatively with a labelfree assay. Labeling assays include fluorescent ligand binding assays, where a fluorescent label is used for quantification, or radioligand binding assays, where a radioactive label is used for detection.

Peptides do not generally penetrate membranes well. Therefore their use in therapy targeting extracellular proteins is advantageous. Like other GPR receptors, GPR56 is a membrane protein that has an extracellular component to which the peptides are proposed to bind, resulting in promotion of downstream signaling events.

Peptides can be synthesized chemically using methods generally known in the art, such as solid phase peptide synthesis (SPPS), originally developed by Merrifield in 1963. In short, SPPS consists of a cycle of steps coupling Fmoc or Boc protected amino acids to a solid polymeric resing and deprotection to liberate free amino groups. The synthesis can be carried out using automatic peptide synthesizers.

The peptides can be chemically modified, for example to enhance solubility or bioavailability, stabilize the peptide or provide auxiliary structure that facilitates the biological activity of the peptide. Exemplary peptide modifications are described in Wang (Signal Transductions and Targeted therapy, 2022, 7:48) and Barman (Internat J Peptide Research Therapeutics 2023 29:61). Modifications include for example protection of N- and/or C-terminus such as Acetylation at the N terminus, amidation at the C terminus; backbone modifications, such as alkylation of at least one nitrogen atom, thionation of at least one carbonyl group and α-carbon modification; glycosylation and PEGylation.

Furthermore, the peptide can additionally or alternatively have a cyclical structure. The peptides can furthermore contain one or more D amino acids, or even consist entirely of D amino acids, to prevent their proteolytic degradation in vivo.

Peptides can be formulated using methods known in the art. Formulation approaches include formulations as emulsions, microspheres, liposomes, nanoparticles and as microparticle based lipids, as described by Naz (PJMHS, 2020, 14:257-263).

Peptides can be delivered using classical routes of administration, including intravenous, intramuscular, subcutaneous administration. Alternate routes are also possible, including transdermal, pulmonary, nasal, oral and buccal routes.

Various delivery systems have been developed to deliver peptide drugs. These include various chemistry and nanophysics-based technologies and formulation methods. Examples include nanocarrier technology, where an inert nano-sized peptide carrier encapsulates and protects the peptide from degradation; examples include micelles and liposomes as nanocarriers; macroflux transdermal technology, where the peptide is carried in a transdermal patch to be delivered in controlled manner; transcytosis methods, combining a ligand to the peptide to assist permeability; PEGylation, where a PEG polymer is attached to the peptide to facilitate blood passage and retaining time, improves circulation and increases stability; depo-foam technology, using liposomal formulations; polymeric micelles as colloidal vehicles; microneedle injections.

The peptides disclosed herein are generally useful for treatment of any subject. Preferably, the subject is human, including pre- and post-pubertal children, adolescents and adults. Preferably, the subject is a human adult.

Treatment methods include a method for treatment of a medical condition as disclosed herein, including a condition selected from vascular dysfunction, cardiovascular disease, impaired pancreatic b-cell function, diabetes, pre-diabetes, metabolic syndrome, depression, Alzheimer's disease, retinopathy and Attention deficit hyperactivity disorder, in a subject in need thereof, the method comprising administering to the subject a therapeutically effective amount of a composition as disclosed herein. The composition can preferably be a pharmaceutical composition, i.e. a composition intended for pharmaceutical use.

The term "treatment" and "treating" as disclosed herein refers to the management and care of a patient for the purpose of combating a condition, such as a disease or a disorder. The term is intended to include the full spectrum of treatments for a given condition from which the patient is suffering, such as administration of the active compound to alleviate the symptoms or complications, to delay the progression of the disease, disorder or condition, to alleviate or relief the symptoms and complications, and/or to cure or eliminate the disease, disorder or condition as well as to prevent the condition, wherein prevention is to be understood as the management and care of a patient for the purpose of combating the disease, condition, or disorder and includes the administration of the active compounds to prevent the onset of the symptoms or complications. The treatment may either be performed in an acute or in a chronic way. The patient to be treated is preferably a mammal, in particular, a human.

The term "a therapeutically effective amount" of the compound for use of the present invention as used herein means an amount sufficient to cure, alleviate or partially arrest the clinical manifestations of a given disease and its complications. An amount adequate to accomplish this is defined as "therapeutically effective amount". Effective amounts for each purpose will depend on the severity of the disease or injury as well as the weight and general state of the subject. It will be understood that determining an appropriate dosage may be achieved using routine experimentation, by constructing a matrix of values and testing different points in the matrix, which is all within the ordinary skills of a trained physician or veterinary.

The disclosure also relates to pharmaceutical compositions comprising compositions as disclosed herein and optionally one or more pharmaceutically acceptable additive, such as a carrier or an excipient.

As used herein "pharmaceutically acceptable additive" is intended without limitation to include carriers, excipients, diluents, adjuvant, colorings, aroma, preservatives etc. that the skilled person would consider using when formulating a compound of the present invention in order to make a pharmaceutical composition.

Adjuvants, diluents, excipients and/or carriers are pharmaceutically acceptable in the sense of being compatible with the compound for use and the other ingredients of the pharmaceutical composition, and not deleterious to the recipient thereof. Suitable adjuvants, diluents, excipients and carriers that may be used in the pharmaceutical composition are well known to a person skilled within the art.

Thus, the compositions and pharmaceutical compositions as disclosed herein may, in addition to the disclosed compositions further comprise at least one pharmaceutically acceptable adjuvant, diluent, excipient and/or carrier. In some embodiments, the pharmaceutical compositions comprise from 1 to 99 % by weight of the at least one pharmaceutically acceptable adjuvant, diluent, excipient and/or carrier and from 1 to 99 % by weight of a composition as disclosed herein.

As used herein, including in the claims, singular forms of terms are to be construed as also including the plural form and vice versa, unless the context indicates otherwise. Thus, it should be noted that as used herein, the singular forms "a," "an," and "the" include plural references unless the context clearly dictates otherwise.

Throughout the description and claims, the terms "comprise", "including", "having", and "contain" and their variations should be understood as meaning "including but not limited to" and are not intended to exclude other components.

The present invention also covers the exact terms, features, values and ranges etc. in case these terms, features, values and ranges etc. are used in conjunction with terms such as about, around, generally, substantially, essentially, at least etc. (i.e., "about 3" shall also cover exactly 3 or "substantially constant" shall also cover exactly constant).

It will be appreciated that variations to the foregoing embodiments of the invention can be made while still falling within the scope of the invention n. Features disclosed in the specification, unless stated otherwise, can be replaced by alternative features serving the same, equivalent or similar purpose. Thus, unless stated otherwise, each feature disclosed represents one example of a generic series of equivalent or similar features.

Use of exemplary language, such as "for instance", "such as", "for example" and the like, is merely intended to better illustrate the invention and does not indicate a limitation on the scope of the invention unless so claimed. Any steps described in the specification may be performed in any order or simultaneously, unless the context clearly indicates otherwise.

All the features and/or steps disclosed in the specification can be combined in any combination, except for combinations where at least some of the features and/or steps are mutually exclusive. In particular, preferred features of the invention are applicable to all aspects of the invention and may be used in any combination.

### EXAMPLES

### Example 1

Peptides were developed based on a structural comparison and analysis of the interaction between GPR56 and collagen type III. The peptide GYYCG was designed *de novo* to bind optimally to the binding site of GPR56 to collagen type III.

The peptides were synthesized using conventional solid phase peptide synthesis.

Peptides NQGCKL and LLSSR were amidated at their respective C-terminal end (i.e., NQGCKL-NH₂ and LLSSR-NH₂.

### Example 2

### Dose-response effect in human pancreatic insulin cells

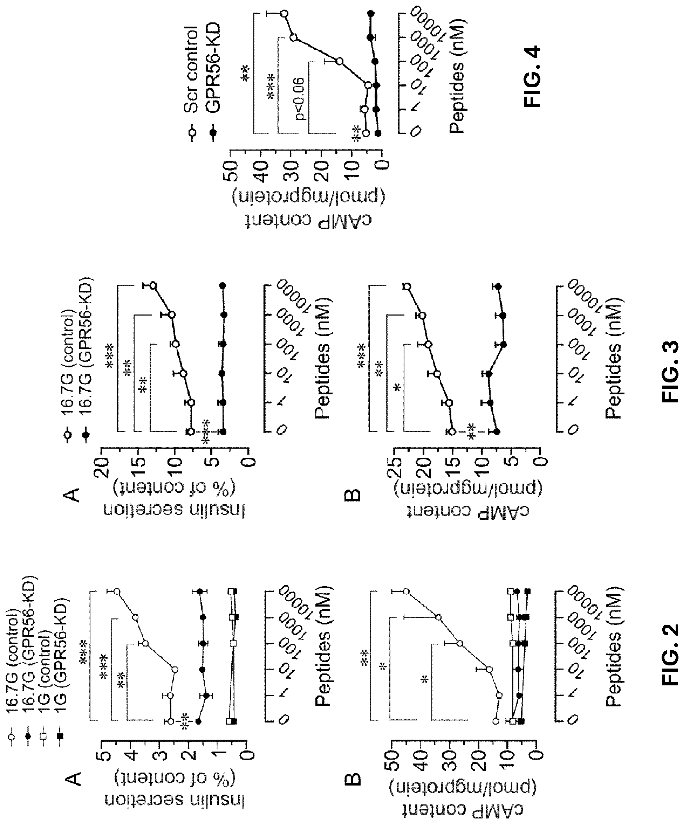

The dose-response effect of synthesized peptides on the insulin secretion (A) and cAMP content in human pancreatic insulin cells (EndoC bH1) cells (B) is shown in FIG. 2. After GPR56-KD, the EndoC βH1 cells were incubated at 1 mM (1G) or 16.7 mM glucose (16.7G) in the presence of increasing concentrations of each peptide in a mixture of the peptides LLSSR, GYYCG and NQGCKL for 60 min. GPR56-KD decreased the glucose-stimulated insulin secretion concomitant with a decreased cAMP content in the EndoC bH1 cells. Presence of peptide-mixture during incubation dose-dependently potentiated glucose-stimulated insulin secretion and cAMP generation. Mean±SD for three experiments performed at three different occasions are shown. *p<0.05, **p<0.01 and ***p<0.001.

### Example 3

### Dose-response effect in rat pancreatic insulin cells

The dose-response effect of the synthesized peptides on the insulin secretion (A) and cAMP content (B) in the rat pancreatic insulin cells (INS-1 832/13) cells was investigated, as shown in FIG. 3. After GPR56-KD, the INS-1 832/13 cells were incubated at 1 mM (1G) or 16.7 mM glucose (16.7G) in the presence of increasing concentrations of the peptide mixture (same as in Example 2) for 60 min. GPR56-KD decreased the glucose-stimulated insulin secretion concomitant with a decreased cAMP content in the INS-1 832/13 cells. Presence of peptide-mixture during incubation dose-dependently potentiated glucose-stimulated insulin secretion and cAMP generation. Mean±SD for four-six experiments performed at four different occasions are shown. *p<0.05, **p<0.01 and ***p<0.001.

### Example 4

*Dose-response effect in human endothelial cells.*

The dose-response effect of the synthesized peptides on the cAMP content in the human endothelial cell line (HUVEC) is shown in FIG. 4. After GPR56-KD, the HUVECs were incubated at 5 mM glucose in the presence of increasing concentrations of the peptide-mixture for 60 min. GPR56-KD decreased the basal cellular cAMP content in the HUVEC cells. Presence of peptide-mixture during incubation dose-dependently potentiated cAMP generation. As control, cells were transfected with scrambled siRNA (Scr control). Mean±SD for four experiments performed at four different occasions are shown. *p<0.05, **p<0.01 and ***p<0.001.

### Example 5

### Effect of peptide agonists on HUVEC cell viability

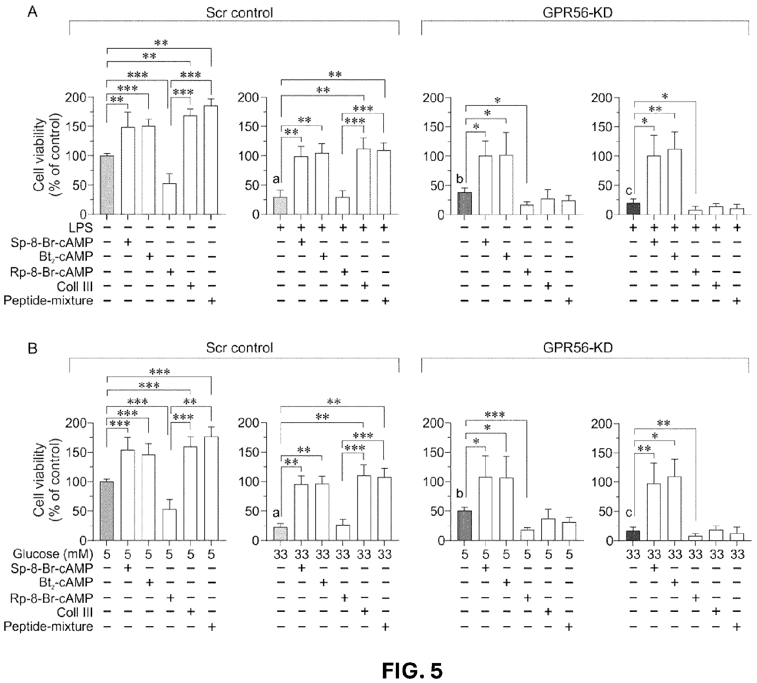

The involvement of PKA in GPR56 mediated restoration of cell function was investigated as shown in FIG. 5.
(A) The HUVEC cells (scramble control and GPR56-KD) were cultured in the presence of PKA activators Sp-8-Br-cAMP and Bt₂-cAMP, PKA inhibitor (Rp-8-Br-cAMP), Coll type III and the peptide mixture (same as in Example 2, each peptide at 1µM) for 24 hours. The cell viability (reductive capacity of the cells) were evaluated by MTS, a colorimetric method of determining cell viability using the MTS tetrazolium compound (3-(4,5-dimethylthiazol-2-yl)-5-(3-carboxymethoxyphenyl)-2-(4-sulfophenyl)-2H-tetrazolium). For calculation, the effect of tested agents were compared to control culture media with 5 mM glucose without presence of any agents, which was kept as 100% in each experiment. Both PKA activators further increased reductive capacity of the cells, whereas PKA inhibitor decreased it. Similar to PKA activators, Coll type III or peptide-mixture also further increased the reductive capacity to the same magnitude. The decreased cell viability in GPR56-KD cells were efficiently counteracted only by PKA activators and not by Coll type III or peptide mixture. PKA inhibitor further reduced the decreased cell viability in GPR56-KD cells. *p<0.05, ** p<0.01 and ***p<0.001.
(B) The HUVEC cells (scramble control and GPR56-KD) were cultured at 5 or 33 mM glucose in the presence of PKA activators Sp-8-Br-cAMP (100 µM) and Bt₂-cAMP (100 µM), PKA inhibitor Rp-8-Br-cAMP (100 µM), Coll type III (20 ug/ml) and the peptide-mixture (1 µM each) for 72 hours. The cell viability (reductive capacity of the cells) were evaluated by MTS. For calculation the effect of tested agents were compared to control culture media with 5 mM glucose (5G) without presence of any agents, which was kept as 100% in each experiment. Both PKA activators (Sp-8-Br-cAMP and Bt₂-cAMP) further increased reductive capacity of the cells, whereas PKA inhibitor (Rp-8-Br-cAMP) decreased it. Similar to PKA activators, Coll type III or peptide-mixture also further increased the reductive capacity to the same magnitude. The decreased cell viability in GPR56-KD cells were efficiently counteracted only by PKA activators and not by Coll type III or peptide mixture. PKA inhibitor further reduced the decreased cell viability in GPR56-KD cells. *p<0.05, ** p<0.01 and ***p<0.001.

In both (A) and (B), a=p<0.001 for effect of LPS (1000 ng/mL) compared to control; b=p<0.001 for the effect of GPR56-KD compared to control; and c=p<0.05 for the effect of GPR56-KD on LPS induced decrease in cell viability (columns in GPR56-KD).

As can be clearly seen, the peptide mixture is able to restore normal cell function that is impaired either by LPS or high glucose treatment.

### Example 6

### Effect of peptide agonists on tube formation in HUVEC cells

Tube formation is a measure of the reassembly of endothelial cells and the establishment of new cell-cell contacts. The cell culture tube formation assay first described in 1988 by Kubota et al. (J Cell Biol, 1988. 107(4): p. 1589-98) is one of the most widely used in vitro assays for angiogenesis. This powerful semiquantitative assay has many advantages. The assay is quick and easy to perform, can be scaled up for high-throughput analysis, and factors can be added exogenously to the medium, transfected into the cells, or knocked down.

GPR56 is essential for tube formation in HUVEC cells, as GPR56-KD abolishes tube formation. To investigate the effect of the peptide mixture on tube formation under glucotoxic conditions, tube formation was observed under normal (5G; equivalent to 5mM) glucose levels, under glucotoxic (20G; equivalent to 20mM) conditions and under normal and glucotoxic conditions in the presence of a peptide mixture.

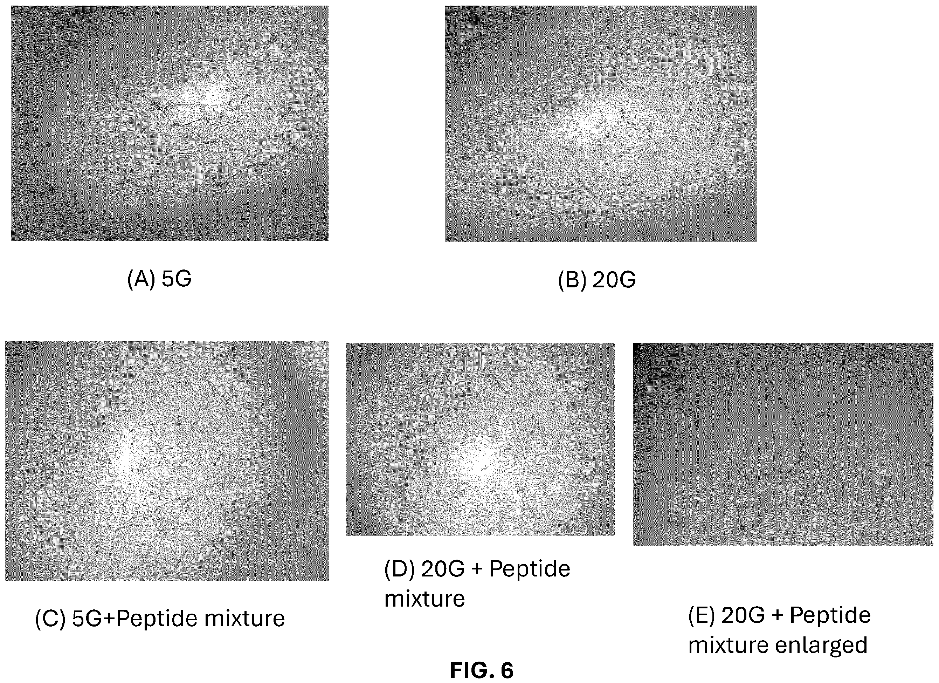

Results are shown in FIG. 6. As can be seen in (A), tube formation is normal in the presence of normal (5G) glucose levels, and the presence of the peptides at this glucose level does not perturb the tube formation as shown in (C). At 20G glucose (B), tube formation is severely impaired, but this impairment is remedied by the presence of the peptide mixture as shown in (D) (see in particular enlarged image in (E)). These results show that the peptides have the capability to rescue endothelial cell function that is lost or impaired under glucotoxic conditions.

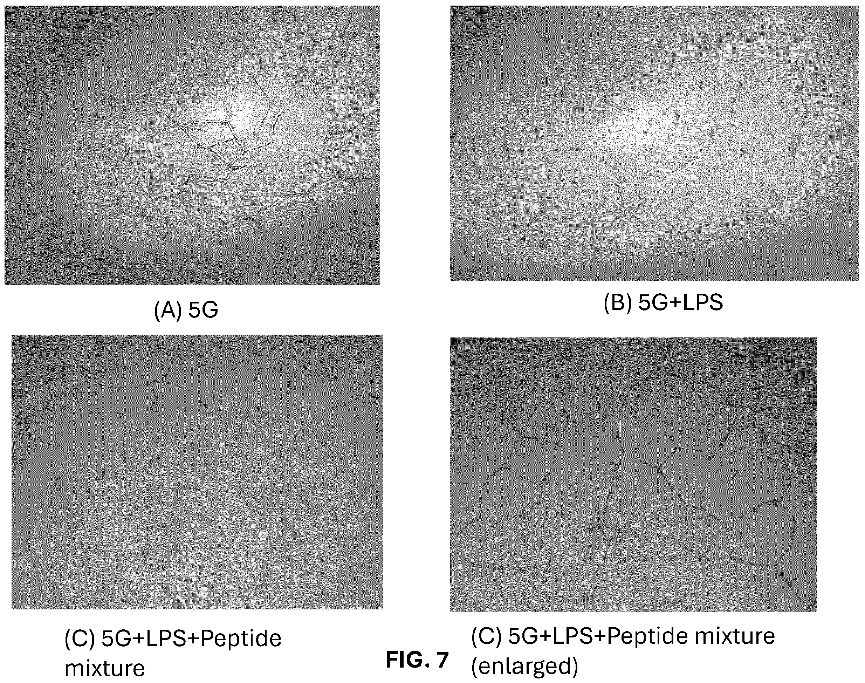

The presence of LPS damages tube formation of HUVEC cells. To investigate the ability of the peptides to rescue this damage, HUVEC cells were incubated in the presence of normal (5G) glucose, in the presence of glucose + LPS, and in the presence of glucose + LPS + peptide mixture. The results, shown in FIG. 7, clearly show that while tube formation, normal at 5G glucose (A), is severely impaired in the presence of 1000 ng/mL LPS (B), the peptide mixture (1µM each peptide) can rescue the cells which show near-normal tube formation, as shown in (C) and (D).

### Example 7.

### Activity of individual peptides and two-peptide mixtures

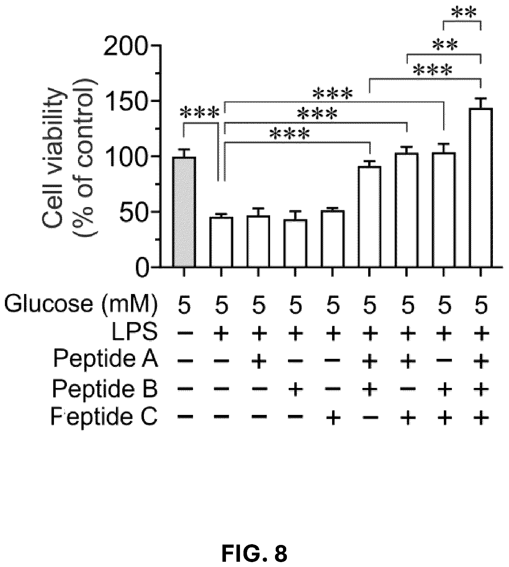

The effects of the peptides GYYCG, NQGCKL and LLSSR, alone or in combinations of were assessed for their effects on cellular viability. The INS-1 832/13 cells were cultured at 5 mM glucose with or without LPS (1000 ng/mL) in the presence of peptide A (LLSSR, 1µM), peptide B (NQGCKL, 1µM) or peptide C (GYYCG, 1µM) alone or in combinations (each peptide 1µM in the combinations) for 24 hours and cellular viability was measured by MTS. Data are Mean±SD from n=3 replicates. ** p<0.01 and ***p<0.0001. As can be seen from the results shown in FIG. 8, any combination of two or three peptides can rescue deficient cellular viability from LPS treatment.

## Claims

1. A composition comprising at least two synthetic or recombinant peptides, each of the peptides comprising a sequence selected from:
LLSSR,
GYYCG, and
NQGCKL,
or a sequence that has a sequence identity of at least 60% thereto, wherein when a peptide comprises sequence a) or b) the peptide has a length of from 5-25 amino acids and wherein when a peptide comprises sequence c) the peptide has a length of from 6-26 amino acids.

2. The composition of claim 1, wherein the at least two peptides are selected from peptides having the general formula:
R₁-X₁X₂SSR- R₂
R₃-X₃YYX₄X₅-R₄, and
R₅-NQGCKX₆-R₆;
wherein
R₁, R₂, R₃, R₄, R₅ and R₆ are independently absent or a peptide sequence of 1-10 amino acids,
X₁, X₂ and X₆ are independently selected from L, A, V, I and G X₃ and X₅ are independently selected from G and A; and
X₄ is selected from G, A and C.

3. The composition of claim 1 or claim 2, wherein the at least two peptides independently optionally comprise at least one modification selected from:
acetylation at the N terminus;
amidation at the C terminus;
alkylation of at least one nitrogen atom;
thionation of at least one carbonyl group;
glycosylation and/or PEGylation; and
wherein the peptides optionally are cyclical and/or contain one or more D-amino acid.

4. The composition according to any one of the previous claims, wherein each of the at least two peptides has a binding affinity to a GPR56 protein to stimulate the activity thereof.

5. A composition according to any one of the preceding claims, wherein the at least two peptides each contains at least one of the sequences LLSSR, GYYCG and NQGCKL.

6. The composition of any one of the preceding claims, the composition comprising at least three peptides that each contain one of the sequences LLSSR, GYYCG and NQGCKL, so that all of the sequences LLSSR, GYYCG and NQGCKL are present in the composition.

7. The composition of any one of the claims 1 - 5, the composition comprising three peptides, each containing one of the sequences LLSSR, GYYCG and NQGCKL.

8. A composition as set forth in any one of the previous claims for use in the treatment of a condition selected from vascular dysfunction, cardiovascular disease, impaired pancreatic β-cell function, diabetes, in particular type II diabetes, impaired glucose tolerance, depression, Alzheimer's disease, retinopathy and Attention deficit hyperactivity disorder (ADHD).

9. A synthetic or recombinant peptide having a length of from 5-26 amino acids and containing a subsequence of at least 5 contiguous amino acid residues from human collagen III (SEQ ID NO:4) for use in the treatment of a condition selected from vascular dysfunction, cardiovascular disease, impaired pancreatic β-cell function diabetes, in particular type II diabetes, impaired glucose tolerance, depression, Alzheimer's disease, retinopathy and Attention deficit hyperactivity disorder.

10. The peptide for use according to the previous claim, wherein the peptide is selected from peptides having the general formula:
R₁-X₁X₂SSR-R_{2;} and
R₅-NQGCKX₆-R₆;
wherein
R₁, R₂, R₅ and R₆ are independently absent or a peptide sequence of 1-10 amino acids; and
X₁, X₂ and X₆ are independently selected from L, A, V, I and G.

11. The peptide for use according to any one of the previous two claims, the peptide containing a sequence selected form NQCGKL and LLSSR.

12. The peptide for use as according to any one of the previous three claims, wherein the peptide has a binding affinity to GPR56 to stimulate the activity thereof.

13. The peptide for use as set forth in any one of the previous four claims, wherein the peptide is selected from:
i. a peptide that is a subsequence of the sequence defined by residues 1282 to 1287 in SEQ ID NO: 4
ii. a peptide that is a subsequence of the sequence defined by by residues 1359 to 1363 in SEQ ID NO:4.

14. The peptide of the previous claim, wherein the peptide in i) contains the sequence NQCGKL and wherein the peptide in ii) contains the sequence LLSSR.

15. A synthetic or recombinant peptide having a length of from 5-20 amino acids and containing at least one sequence selected from:
LLSSR,
GYYCG, and
NQGCKL,
or a sequence that has a sequence identity of at least 60% thereto, for use in the treatment of a condition selected from vascular dysfunction, cardiovascular disease, impaired pancreatic β-cell function, diabetes, in particular type II diabetes, impaired glucose tolerance, depression, Alzheimer's disease, retinopathy and Attention deficit hyperactivity disorder.
